# EUROPEAN PATENT SPECIFICATION

(11) **EP 2 041 576 B1**
(45) Date of publication and mention of the grant of the patent: **10.08.2011**
(21) Application number: 07764767.5
(22) Date of filing: 21.06.2007
(51) Int. Cl.: G01N 33/574

(54) **DIFFERENTIAL CYTOKINE EXPRESSION IN HUMAN CANCER**
DIFFERENTIELLE CYTOKINEXPRESSION BEI EINER MENSCHLICHEN KREBSERKRANKUNG
EXPRESSION DIFFÉRENTIELLE DE LA CYTOKINE DANS LES CANCERS CHEZ L'HOMME

(30) Priority: 21.06.2006 EP 06012754
(43) Date of publication of application: 01.04.2009
(73) Proprietor: Apogenix GmbH, 69120 Heidelberg (DE)
(72) Inventor: STASSI, Giorgio, I-90151 Palermo (IT); GIEFFERS, Christian, 69221 Dossenheim (DE); HILL, Oliver, 69239 Neckarsteinach (DE); THIEMANN, Meinolf, 69198 Schriesheim (DE); TODARO, Matilde, I-90151 Palermo (IT)
(74) Representative: Weiss, Wolfgang
(86) International application number: PCT/EP2007/005480
(87) International publication number: WO 2007/147600

(56) References cited:
- WO-A-96/04388
- US-B1- 6 548 655
- HASSUNEH M R ET AL: "Evidence for the participation of interleukin-2 (IL-2) and IL-4 in the regulation of autonomous growth and tumorigenesis of transformed cells of lymphoid origin" BLOOD, W.B.SAUNDERS COMPANY, ORLANDO, FL, US, vol. 89, no. 2, 15 January 1997 (1997-01-15), pages 610-620, XP002434569 ISSN: 0006-4971
- ALAS S ET AL: "Inhibition of interleukin 10 by rituximab results in down-regulation of bcl-2 and sensitization of B-cell non-Hodgkin's lymphoma to apoptosis." CLINICAL CANCER RESEARCH : AN OFFICIAL JOURNAL OF THE AMERICAN ASSOCIATION FOR CANCER RESEARCH MAR 2001, vol. 7, no. 3, March 2001 (2001-03), pages 709-723, XP002454245 ISSN: 1078-0432
- CZARNESKI J ET AL: "Studies in NZB IL-10 knockout mice of the requirement of IL-10 for progression of B-cell lymphoma." LEUKEMIA (BASINGSTOKE), vol. 18, no. 3, March 2004 (2004-03), pages 597-606, XP002454246 ISSN: 0887-6924
- KRUSE SUSANNE ET AL: "Characterization of the membrane-bound and a soluble form of human IL-4 receptor alpha produced by alternative splicing" INTERNATIONAL IMMUNOLOGY, vol. 11, no. 12, December 1999 (1999-12), pages 1965-1969, XP002462085 ISSN: 0953-8178
- BORISH L C ET AL: "INTERLEUKIN-4 RECEPTOR IN MODERATE ATOPIC ASTHMA A PHASE I/II RANDOMIZED, PLACEBO-CONTROLLED TRIAL" AMERICAN JOURNAL OF RESPIRATORY AND CRITICAL CARE MEDICINE, AMERICAN LUNG ASSOCIATION, NEW YORK, NY, US, vol. 160, 1999, pages 1816-1823, XP002908844 ISSN: 1073-449X
- [Online] Retrieved from the Internet: <URL:http://gateway.nlm.nih.gov/meetingsAbs tracts/ma?f=102261284.html>
- [Online] Retrieved from the Internet: <URL:www.rocklandhemonc.com/provided_servic es.html>
- [Online] Retrieved from the Internet: <URL:www.clinicalconnection.com/exp/expande dpateintviewstudy651696.aspx>
- [Online] Retrieved from the Internet: <URL:www.cancerlinkusa.com/cancer/young/sol idtumor.htr>
- Parhmam, P.: 2005, Garland Science ISBN: 0-8153-4093-1 page 414,
- 'Pschyrembel', ISBN 3110176211 page 2004

## Description

The invention concerns a method for diagnosing a cancer type, whereby the expression of anti-apoptotic cytokines in the tumour cells is determined. The differential diagnosis of the present invention is used to classify tumour disorders and to recommend the required treatment and to monitor the progress and response to the treatment.

The balance between cell survival and cell death is controlled by pro-apoptotic and anti-apoptotic factors, whose dysregulation contributes to the development of several pathological conditions, including cancer. High expression of anti-apoptotic factors is commonly found in human cancers and contributes to both neoplastic cell expansion and resistance to the therapeutic action of cytotoxic drugs. It has already been reported that autocrine production of anti-apoptotic cytokines by tumour cells strongly modulates the susceptibility to the receptor and chemotherapy-induced apoptosis. In particular, it has previously been reported that IL-4 and IL-10 act as autocrine growth factor in cancer cells inducing upregulation of anti-apoptotic proteins, which protect the tumour cells from the death induced by chemotherapeutic drugs (Stassi et al., Cancer Res. 63, 6784-90 (2003), Todaro et al., Cancer Res. 66, 1491-9 (2006)).

Tumours are composed of a heterogeneous combination of cells, with different therapeutic characteristics and different proliferative potentials. In particular, cancer cells may give rise to phenotypically diverse progeny of cells, either endowed with a definite proliferative potential or having a limited or no proliferative potential.

In this respect recent evidence suggests that the tumourigenic growth capacity is in fact confined to a small subset of so-called cancer stem cells (CSC). The International Application PCT/IT2005/000523 discloses a method for isolation and culturing of stem cells from solid tumours. This subpopulation of cancer cells can self-renew and give rise to a population of heterogeneous cells which exhibit diverse degrees of differentiation. Moreover, it has recently been found that these cancer stem cells are significantly resistant to drug-induced apoptosis, thus escaping anti-tumour therapies and this being probably the underlying reason for chemotherapy inefficiency.

Hassuneh et al. (Blood, vol. 89, pp. 610-320, 1997) disclose evidence for the participation of IL-2 and IL-4 in the regulation of autonomous growth and tumorigenesis of transformed cells of lymphoid origin.

Alas et al. (Clin. Canc. Res. Vol. 7, pp. 709-723, 2001) relates to the inhibition of IL-10 by Rituximab resulting in down-regulation of Bcl-2 and sensitization of B-cell Non-Hodgkin's Lymphoma to Apoptosis.

Czarneski et al. (Leukemia, Vol. 18, pp. 597-606, 2004) describe studies in NZB IL-10 knockout mice of the requirement of IL-10 for progression of B-cell lymphoma.

US Patent No. 6,548,655 refers to IL-4 receptors IL-4R and administration of soluble IL-4R, with clinical applications being allergy-therapy and transplantation therapy, as well as suppression of delayed-type or contact hypersensitivity.

WO 96/04388 relates to antagonists of human IL-4 and/or IL-13 for the treatment of e.g. IgE-mediated allergic diseases.

Kruse et al. (Int. Immunol., vol. 11, pp. 1965-1969, 1999) describe the characterization of the membrane-bound and a soluble form of human IL-4 receptor a produced by alternative splicing.

Borish et al. (Am. J. Respir. Crit. Care Med., vol. 160, pp. 1816-1823, 1999) refer to a clinical trial regarding IL-4 receptor in moderate atopic asthma.

It has now been demonstrated that CSC predominantly produce IL-4 and IL-10 and are responsible for the above mentioned alteration of sensibility to drug-induced cell death.

It has now also been found by the inventors of the present invention that epithelial tumours may be differentiated in respect of anti-apoptotic cytokine expression level and/or profile. The expression of anti-apoptotic cytokines differs between individual tumours of the same organ and even within cells or portions of a single tumour. These results lead to new efficient strategies in the tumour diagnosis and/or therapy.

In particular, an object of the present invention was to provide a method which allows the identification and diagnosis of cancer types and cancer cells which express anti-apoptotic cytokines.

The present invention is defined by the appended set of claims.

Accordingly, the present invention provides a method for diagnosing epithelial tumour types, using the anti-apoptotic cytokines IL-4 and/or IL-10 as a target. Particularly, the invention refers to a method for diagnosing a cancer type according to claim 1.

Hence, the invention concerns the differential diagnosis of cancer types by means of the determination and/or quantification of the expression profile and/or level of anti-apoptotic cytokines in the tumour sample, wherein the anti-apoptotic cytokines are IL-4 and/or IL-10, particularly IL-4. Anti-apoptotic cytokines herein are, if not further specified, IL-4 and/or IL-10.

The differential diagnosis according to the invention allows to classify tumour types and to identify those which show expression of anti-apoptotic cytokines and which are refractory to treatment with chemotherapeutic agents. Hence, the expression of anti-apoptotic cytokines is a significant marker for tumour classification which allows a selection of targeted therapeutic strategies.

For example, the method of the invention may be useful to predict whether a patient suffering from a certain cancer type would be resistant or susceptible to a certain therapy and to provide an optimised treatment strategy.

According to the present invention, it was found that cancer types can be classified as non-cytokine-expressing tumours or as cytokine-expressing tumours.

When determining the expression of IL-4 and/or IL-10, more particularly, the expression of IL-4, the epithelial tumours are classified with regard to their expression of either only IL-4 or only IL-10 or both IL-4 and IL-10. Therefore, the method according to the present invention allows the differentiation between epithelial tumour classified as IL-4-expressing tumours or IL-4 non-expressing tumours, epithelial tumour classified as IL-10-expressing tumours or IL-10 non-expressing tumours and epithelial tumour classified as IL-4 and IL-10-expressing tumours or non-IL-4 and non-IL10 expressing tumours.

The method of the present invention is preferably performed on epithelial tumours. Said epithelial tumours may be chosen from the group consisting of thyroid, breast, prostate, bladder, colon, gastric, pancreas, kidney, liver and lung cancer. More preferably, the epithelial tumour is a colon, gastric, breast, lung, bladder or prostate cancer.

The diagnostic method of the present invention may be performed on various cell samples from an epithelial tumour. The test sample is preferably a cell sample from primary tumour and/or from the tumour environment isolated from a subject, e.g. a human patient. For example, tumour cell tissue obtained by biopsy, resection or other techniques can be tested. The tumour sample comprises tumour cells. The expression of anti-apoptotic cytokine in the tumour cells is preferably determined on primary tumour cells and/or cancer stem cells.

Methods for the determination of the anti-apoptotic cytokine expression in the tumour cells are well-known in the art. The determination of the expression, in particular of the overexpression, of the anti-apoptotic cytokine in the tumour cell is conducted by the detection of said cytokine on the protein level and/or the nucleic acid level.

The determination of cytokine proteins may be performed in the tumour cells or in the tumour microenvironment. Methods to determine the presence and amount of cytokine proteins in a given sample are well known to the person skilled in the art and may be immunochemical methods such as immunohistochemistry, Western blotting, immunoprecipitation and ELISA methods. Further methods based on massspectrometry, comprising MALDI-MS, can be used to determine presence and amount of cytokine proteins.

Cytokine nucleic acids are detected and quantified herein by any of means well known to those skilled in the art. Hybridization techniques together with optional amplification methods are frequently used for detecting nucleic acids. Expression of cytokine mRNAs may for example be detected by Northern blot analysis or by reverse transcription and subsequent amplification by PCR.

The method according to the invention may comprise the further step of
(d) determining the sensitivity of the cells of a cytokine expressing tumour against at least one chemotherapeutic or pro-apoptotic agent in the presence and/or in the absence of an antagonist of said expressed cytokine and/or its receptor.

In order to investigate the sensitivity of the cytokine-expressing tumour cells to chemotherapeutic and/or pro-apoptotic agents, the viability of the tumour cells exposed to said chemotherapeutic or pro-apoptotic agents in the absence and/or presence of cytokine neutralizing agents may be measured. Methods for determining the sensitivity of the tumour cells to a given agent are well known by those skilled in the art (e.g. as described in Examples).

Based on this determination, the method according to the present invention may further comprise the step of
(e) selecting a cancer type-specific treatment.

As already mentioned, the invention is based on the observation that epithelial tumours may be differentiated by their expression or degree of expression of anti-apoptotic cytokines and in particular IL-4 and IL-10 cytokines. Since the expression of IL-4 and IL-10 anti-apoptotic cytokines in tumours or tumour cells is responsible for refractoriness to treatment, e.g. with chemotherapeutic and/or pro-apoptotic agents, the anti-apoptotic cytokines should be neutralized in order to increase the sensitivity of the tumour towards treatment. Thus, the invention may also encompass an examination of the sensitivity or resistance to chemotherapeutic and/or pro-apoptotic agents in combination with antagonists of a cytokine expressed by the tumour.

In a preferred embodiment, the sensitivity assay performed in step (d) of the method leads to the determination of a chemotherapeutic or a pro-apoptotic agent against which the cell of the cytokine-expressing tumours are particularly sensitive.

Consequently, according to step (e) of the present invention, a successful tumour type-specific treatment may be selected comprising the administration of a combination of a cytokine-neutralizing agent, namely a IL-4 cytokine neutralizing agent and a chemotherapeutic or pro-apoptotic agent, which is TRAIL or CD95L.

A cytokine-neutralizing agent may be any compound which reduces the amount and/or activity of a cytokine. For example, the cytokine neutralizing agent may be an agent which inhibits a signal transduction pathway triggered by the cytokine autocrinely expressed by the tumour cells. Hence, any agent is contemplated that is capable of modulating the expression and/or function of a cytokine directly and/or indirectly, namely affecting the expression and/or function of the respective cytokine protein and/or cytokine receptor.

The cytokine neutralizing agent is an IL-4 neutralizing agent, i.e. any agent which is able to inhibit the signal transduction pathway triggered by the autocrine expression of IL-4.

Cytokine neutralizing agent is selected from agents that inhibit the expressed cytokine protein activity. Agents that block the cytokine activity are, for example, antagonists which block the cytokine receptors, e.g. peptides, small molecules, muteine variants of the cytokines which show an antagonistic activity compared to the original signal of the cytokine. Examples for such muteins are in particular IL-4 muteins such as Aerolast^{®} from Aerovance and Pitrakinra^{®} and BAY-36-1677 from Bayer. Further antibodies against the cytokine receptor or antibodies against the cytokine protein may be used. The antibody is preferably an antibody against IL-4 and/or IL-10, e.g. antibodies from Amgen and Immunex or an antibody against the IL-4 receptor and/or the IL-10 receptor, e.g. the antibody Pascolizumab^{®} from Glaxo. The antibody may be a complete antibody, e.g. an IgG antibody, or an antigen-binding fragment thereof. Preferably, the antibody is a monoclonal chimeric or humanized antibody which has human constant domains, e.g. human constant IgG1, IgG2, IgG3 or IgG4 domains. More preferably, the antibody is a humanized antibody which additionally comprises human framework regions. Also preferred are antibody fragments, e.g. divalent or monovalent antibody fragments such as F(ab)₂ fragments. On the other hand, the antibody may be a recombinant antibody, e.g. a single chain antibody or a fragment thereof, e.g. an scFv fragment.

Soluble cytokine receptors, preferably without the membrane spanning and the intracellular domain, can also be used as agents blocking the cytokine activity. These soluble receptors are, for example, from Regeneron, in particular IL-4R/IL-13R-Fc fusion proteins, and soluble receptors from Amgen and Immunex, in particular Nuvance^{®} and Altrakincept^{®}. Specific examples of soluble receptors comprise the extracellular domain (ECD) of a human IL-4 receptor, e.g. from a shortened ECD of human IL-4R alpha amino acid 24 to amino acid 224, 225, 226, 227, 228, 229 or 230 and optionally further domains, e.g. the extracellular domain of a human II-13 receptor and/or a human Fc immunoglobulin domain.

As preferred example of agents that degrade the expressed cytokine protein designer proteases can be mentioned in the context of the present invention. The production of the cytokine proteins can, on the other hand, be inhibited for example by agents acting on the nucleic levels such as antisense nucleic acids, siRNA molecules and/or ribozymes.

Preferred cytokine antagonists are described in the international patent application WO 2004/069274. Antibodies directed against cytokines are preferably used as cytokine-neutralizing agents. Anti-IL-4 antibodies disclosed in European patent application EP-A-0 730 609 are especially suitable as cytokine-neutralizing agents of the method of the present invention. In a very preferred embodiment, the antibody derived from the monoclonal antibody 6A1 produced by hybridoma cell line ACC93100620 or an antigen-binding fragment thereof is used as cytokine-neutralizing agent.

The chemotherapeutic agent used in steps (d) and/or (e) is selected from antimetabolites, DNA-fragmenting agents, DNA-cross-linking agents, intercalating agents, protein synthesis inhibitors, topoisomerase I and II inhibitors, micro-tubule-directed agents, kinase inhibitors, hormones and hormone antagonists. Particularly, the chemotherapeutic agent is selected from cisplatin, carboplatin and oxaliplatin. Pro-apoptotic agents are TRAIL or CD95 ligand.

Based on the results obtained from the combined administration of anti-therapeutic cytokine-antagonists and chemotherapeutic and/or pro-apoptotic agents to the tumour cell, a therapeutic strategy can be developed based on a specific combination of drugs which has proven to be effective.

A further object of the present invention is therefore the use of a combination of a cytokine-neutralizing agent and a chemotherapeutic or pro-apoptotic agent and the manufacture of a medicament for the tumour treatment, such as a first line tumour treatment or as second or third line tumour treatment, e.g. for the treatment of refractory tumours, such as tumours which have become refractory against one or more anti-tumour agents.

Thus, a further aspect of the present invention is the use of a combination of
(i) at least one IL-4 cytokine-neutralizing agent and
(ii) at least a chemotherapeutic or pro-apoptotic agent which is TRAIL or CD95L
for the manufacture of a medicament for the treatment of a cancer type classified as cytokine-expressing epithelial tumour.

One of the main causes of drug resistance in tumour cells is based on the observation that a surviving small population of tumour cells, and in particular of tumour stem cells, after an apparently complete regression or surgical excision of the primary tumour could renew the tumour and contribute to the so called minimal residual disease (MRD).

In this respect, since the combination therapy is particularly suitable for increasing the therapeutic sensitivity of tumour stem cells. A further aspect of the present invention is the use of a combination of
(iii) at least one IL-4 cytokine-neutralizing agent and
(iv) at least a chemotherapeutic or pro-apoptotic agent which is TRAIL or CD95L
for the manufacture of a medicament for the treatment of epithelial tumors that have become refractory to chemotherapeutic agents.

According to a preferred embodiment of the present invention, the use of a combined therapy of the above agents (i) and (ii) can further be in combination with surgery and/or irradiation therapy. In particular, the medicament combination is for simultaneous, separate or sequential combination therapy with surgery and/or irradiation therapy.

According to one preferred embodiment of the present invention, the administration of agent (i) and agent (ii) is started simultaneously. Alternatively, the combination therapy can be started stepwise. According to this preferred embodiment of the invention, the start of the administration of the cytokine-neutralizing agent (i) is ≤ 1 week before the administration of the chemotherapeutic or pro-apoptotic agent (ii). The administration of the chemotherapeutic or pro-apoptotic agent (ii) may in turn start ≥ 1 week before the administration of the cytokine-neutralizing agent (i).

Still a further embodiment of the invention is a soluble human IL-4 receptor polypeptide which is derived from human IL-4 receptor alpha (NCBI accession NP_000409), which C-terminally ends at amino acid 230, 229, 228, 227, 226, 225 or 224 for use in a method for the treatment of epithelial tumors. Preferably the C-terminal end is amino acid 224. The polypeptide may comprise at least one further domain, e.g. an N-terminal signal peptide, a further effector domain, e.g. an IL-13 receptor extracellular domain, an Fc immunoglobulin domain, and/or a purification domain. An example of a shortened IL-4R polypeptide is described in Example 4. The shortened IL-4R polypeptide is suitable for pharmaceutical applications, e.g. for the treatment of tumours, particularly for the treatment of IL-4-associated tumours as described above. hrs. Anti-CD95 (mAb CH-11, IgM; Upstate Biotechnology Inc.) or control IgM (Sigma) or isoleucine zipper TRAIL (iz-TRAIL; 200 ng/ml) were used to determine sensitivity to CD95- or TRAIL-induced apoptosis in cancer cells. Moreover, following exposure to anti-IL-4 and anti-IL-10 cancer cells were treated with oxaliplatin (100 µM) or doxorubicin (5 µM) or cisplatin (300 ng/ml), or taxol (5 µM) (Sigma) or etoposide (1 µM; Biomol, Plymouth Meeting, PA).

**Survival and death assays.** To evaluate apoptotic events the DNA staining and flow cytometry analysis were performed. The percentage of hypodiploid nuclei was evaluated as described in Stassi et al., Cancer Res. 2003, 63 (20):6784-90. Alternatively, human purified cancer cells were plated in 96-well plates in triplicate at 15,000 cells/well and cultured. The number of viable cells was detected by CellTiter Aqueous Assay Kit (Promega Corporation, WI, USA) following the instructions of manufacturer. HuT78 cells plated at 2 x 150/ml and treated with CD95-activating antibody CH11 (200 ng/ml) were used as a positive control for cell death measurement.

**Immunohistochemical analysis.** Immunohistochemistry was performed on 5 µm thick paraffin-embedded colon, gastric, prostate, breast, lung, liver, pancreas, kidney and bladder normal and tumour sample sections. Dewaxed sections were treated for 10 min in microwave oven in 0.1 M citrate buffer. Then, sections were incubated for 10 min with Tris Buffer Saline (TBS) containing 10% AB human serum to block the unspecific staining. After elimination of excess serum, sections were exposed overnight at 4°C to specific antibodies against IL-4 (B-S4 mouse IgG1, Caltag Laboratories, Burlingame, CA), IL-10 (B-N10 mouse IgG₂ₐ, Caltag), IL-4Rα (C-20 rabbit IgG Santa Cruz Biotechnology Inc, Santa Cruz, CA), IL-10R (C-20 rabbit IgG Santa Cruz Biotechnology), TRAIL-R1 (HS101 mouse IgG1, Alexis Biochemicals, Lausen, CH) TRAIL-R2 (HS201 mouse IgG1, Alexis) or isotype-matched controls at appropriate dilutions. Following exposure to primary antibody cells were treated with biotinylated anti-rabbit or antimouse immunoglobulins, washed in TBS and then incubated with streptavidin peroxidase (Dako LSAB 2 Kit, Dako Corporation Carpinteria CA, USA). Staining was detected using 3-amino-9-ethylcarbazole (AEC) as a colorimetric substrate. Counterstaining of cells was performed using aqueous hematoxylin.

**RT-PCR analysis.** Total RNA was prepared from cultured cells using the Rneasy Mini Kit (Qiagen GmbH, Germany) according to manufacturer's instructions. Reverse transcription and PCR amplification for each preparation with 1µg of total RNA was performed using OneStep RT-PCR Kit (Qiagen). Two primers specific for the IL-4 coding sequence 5'-CCA CGG ACA CAA GTG CGA TA nucleotides 436-455 (exon 1) and 5'-CCT TGC AGA AGG TTT CCT TCT-3' complementary to nucleotides 564-584 (exon 3) (GenBank accession number NM 000589.2) were selected to specifically amplify IL-4.

GAPD gene was amplified from the same RNA preparations as housekeeping control (coding sequence 5'-TGA CAT CAA GAA GGT GGT GA-3' nucleotides 843-863 and 5'-TCC ACC ACC CTG TTG CTG TA-3' complementary to nucleotides 1033-1053; NM-002046 accession number). Thirty-five cycles were performed, each consisting of the following conditions: 94°C, 30 sec; 58°C, 30 sec; 72°C, 30 sec.

**Protein isolation and western blotting analysis.** Cell pellets were resuspended in ice-cold NP-40 lysis buffer (50 mM Tris-HCl, pH 7.5, 150 mM NaCl, 1 mM EGTA, 1% NP-40) containing protease inhibitors as described in Stassi et al. Nature Immunology 2000, 1, 1-6. Immunoblotting of Abs specific for actin (Ab-1, mouse IgM, Calbiochem, Darmstadt, Germany), CD95L (G247-4, mouse IgG1, PharMingen, San Diego), CD95 (C-20, Santa Cruz Biotechnology), cFLIP (NF6 mouse IgG1, Alexis Biochemicals, Switzerland), PED/PEA-15 (rabbit IgG kindly provided by G. Condorelli), Bcl-2 (124, mouse IgG1, Upstate Biotechnology Inc.) and Bcl-X_{I} (H-5, mouse IgG1, Santa Cruz Biotechnology) was detected by HRP-conjugated antimouse or anti-rabbit Abs (Amersham Biosciences UK Limited, England) and visualized with the chemiluminescence detection system (SuperSignal West Dura Extended duration Sustrate, Pierce, Illinois, USA).

### Example 1

### Autocrine production of IL-4 in cancer cells

In order to investigate if the tumour microenvironment influences cancer cell phenotype and function, the presence of IL-4 and IL-10 previously found to be autocrinely produced by cancer thyrocytes was evaluated. Immunohistochemistry analyses demonstrated that all the investigated solid tumour histotypes expressed high levels of IL-4, while IL-10 was less detectable. Results are shown in Table 1.

**Table 1. Cytokine expression in cancer cells**

| **Cancer** | **IL-4** | **IL-10** |
|---|---|---|
| PTC | ++++ | +++ |
| FTC | ++++ | +++ |
| UTC | ++++ | ++++ |
| Colon | ++++ | + |
| Gastric | +++++ | - |
| Lung | ++++ | + |
| Pancreas | + | + |
| Glioblastoma | +++ | ++ |
| Prostate | ++ | + |
| Breast | ++++ | + |
| Bladder | ++++ | + |
| Liver | + | + |
| Kidney | ++ | ++ |

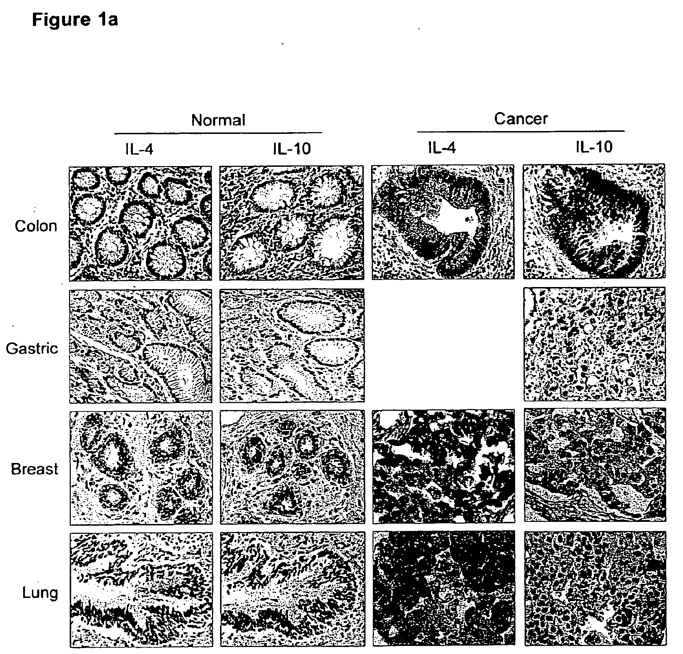

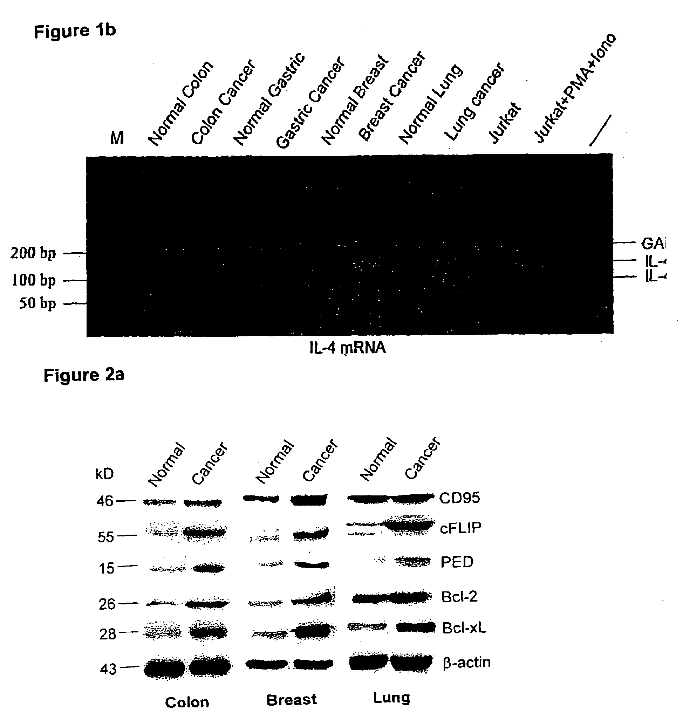

Interestingly, the reactivity against IL-4 localized to colon, breast, lung, gastric, liver, prostate, pancreas, kidney and bladder cancer cells, suggesting that neoplastic cells are the source of high production for IL-4 and less for IL-10 (Table 1 and Fig. 1a). To exclude the possibility that the reactivity observed in tumour cells was exclusively due to the release of type 2 cytokines by infiltrating T cells, freshly purified colon, breast, gastric and lung cancer cells were analyzed by RT-PCR. In agreement with immunohistochemistry results, IL-4 mRNA expression levels of purified cancer cells were highly increased compared to related normal cells (Fig. 1b), demonstrating that autocrine production of IL-4 is not restricted to thyroid cancer cells but also takes place in other epithelial malignant cells from solid tumours which produce considerable amounts of IL-4.

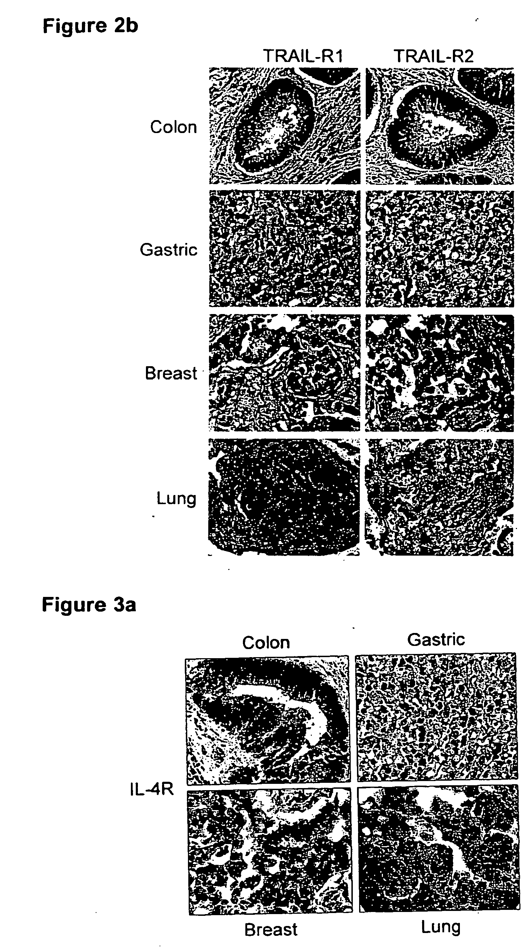

**Epithelial cancer cells express high levels of anti-apoptotic proteins.** Colon, breast, gastric and lung cancer cells are resistant to death ligand- and to chemotherapy-induced cell death. To determine the mechanism responsible for this refractoriness, it was investigated whether aberrant expression of anti-apoptotic factors could be implicated in the impaired "extrinsic" and "intrinsic" apoptotic signal pathway generated by death ligands or chemotherapy. It was found by immunohistochemistry and Western blot analyses that epithelial carcinoma cells express CD95, TRAIL-R1 and TRAIL-R2 (Fig. 2a and b). Therefore, the inventors of the present invention evaluated the presence and measured the expression levels of cFLIP, PED/PEA-15, Bcl-xL and Bcl-2 in colon, breast, gastric and lung normal and cancer cells. While cFLIP and PED/PEA-15 levels were approximately three fold higher in freshly purified cancer cells, as compared with normal colon, breast and lung cells (Fig. 2a), Bcl-xL levels were four fold higher. Bcl-2 expression levels were only two fold higher in all the cancer cells analyzed, as compared with normal cells. Thus, anti-apoptotic genes upregulation in colon, breast, gastric and lung cancer cells may confer resistance to CD95- TRAIL- and chemotherapy-induced apoptosis.

### IL-4 increases survival, growth of epithelial neoplastic cells.

The expression of IL-4 receptor in both normal and neoplastic cells was evaluated. Immunohistochemistry on paraffin embedded sections showed that IL-4 receptor was expressed in all the cancer tissues analysed. The results are shown in the following Table 2 and in Fig. 3a.

**Table 2. IL-4R expression in cancer cells**

| **Cancer** | **IL-4R** |
|---|---|
| PTC | ++++ |
| FTC | ++ |
| UTC | +++ |
| Colon | ++ |
| Gastric | +++ |
| Lung | +++++ |
| Pancreas | +++ |
| Glioblastoma | ++ |
| Prostate | ++ |
| Breast | +++ |
| Bladder | ++++ |
| Liver | +++ |
| Kidney | +++ |

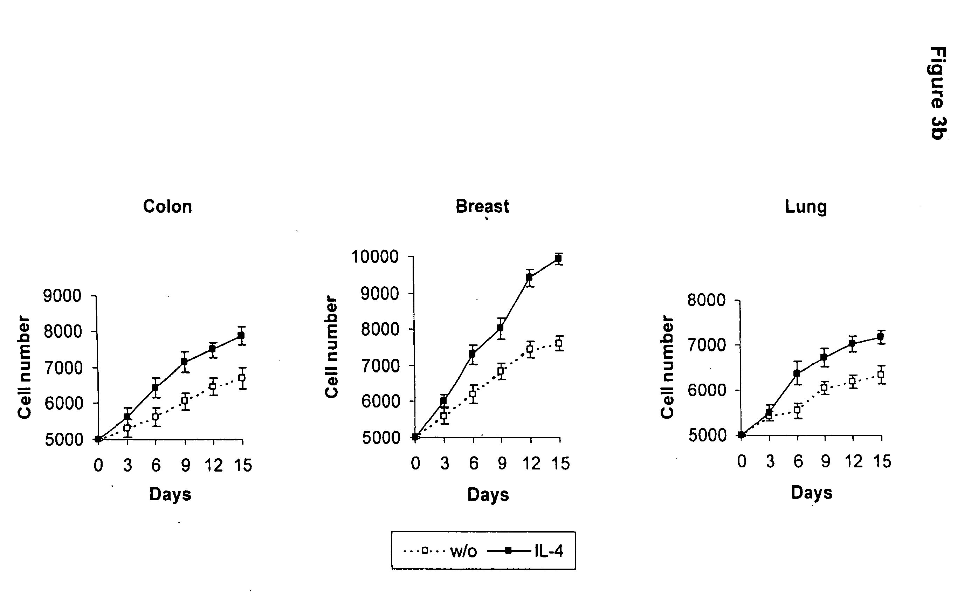

In order to investigate the possible involvement of IL-4 on tumour cell survival, colon, breast, gastric and lung normal cells were exposed to 20 ng/ml of IL-4 and analyzed for cell growth. IL-4 significantly increased the growth rate of colon, breast and lung normal cells (Fig. 3b).

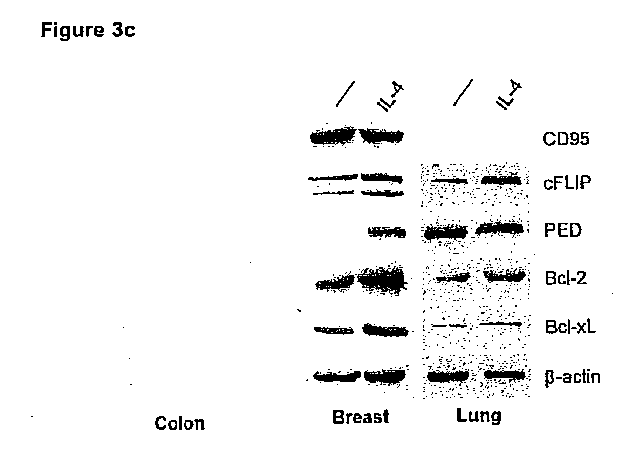

Furthermore, to determine the involvement of IL-4 in the refractoriness of cancer cells to CD95, TRAIL and chemotherapeutic agents, normal colon, breast, gastric and lung cells were pre-incubated with IL-4 and then analyzed for expression of those anti-apoptotic proteins implicated in the death ligands and chemotherapy cell death resistance. IL-4 increased the protein levels of cFLIP, PED/PEA-15, Bcl-xL and Bcl-2 in normal colon, breast (Fig. 3c) and gastric and lung cells, suggesting that autocrine IL-4 production might protect cancer cells from chemotherapy and death receptor stimulation, up regulating anti-apoptotic factors.

### IL-4 neutralization promotes growth arrest and cell death induced by CD95, TRAIL and chemotherapy in cancer cells

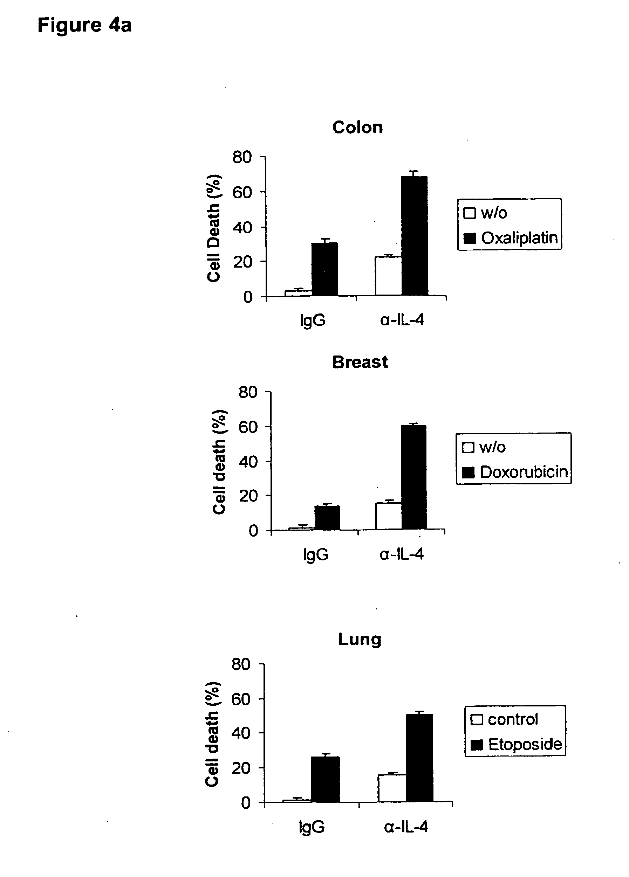

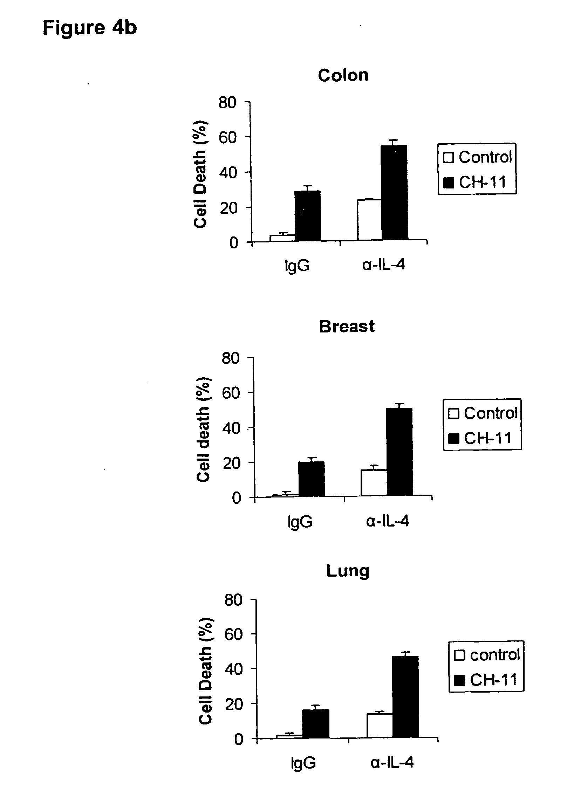

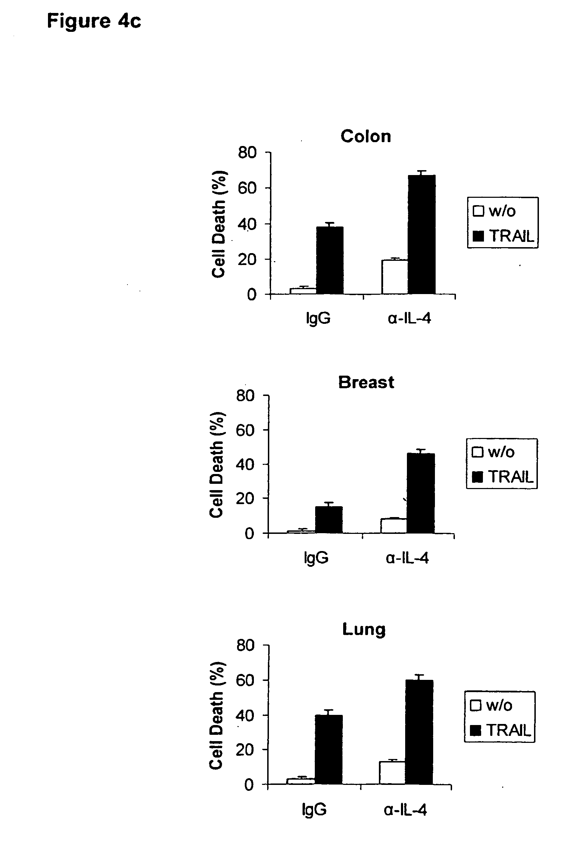

To directly demonstrate that autocrine production of IL-4 confers protection from cell death induced by CD95, TRAIL and chemotherapy, we investigated the effects of IL-4 neutralization in colon, breast and lung cancer cells. Exposure of freshly purified colon, breast, gastric and lung cancer cells to neutralizing antibodies against IL-4 for 48 hrs sensitized cancer cells to chemotherapy- and death receptor-induced cell death confirming the anti-apoptotic role of IL-4 in solid cancer. The results are shown in the Figures 4a-c.

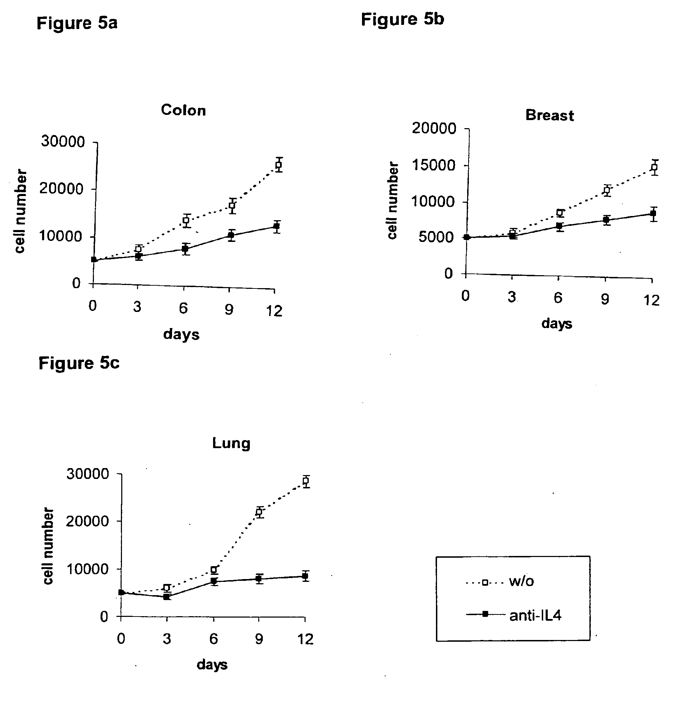

Furthermore, IL-4 neutralization blocked colon, breast, gastric and lung tumour cell growth up to 15 days (Fig. 5) and down-modulated the protein expression levels of cFLIP, PED/PEA-15, Bcl-xL and Bcl-2. These data indicate that autocrine production of IL-4 might play an important role in growth control and is specifically required for survival of cancer cells.

Tissue specimens from freshly operated tumour patients were screened for IL-4 and IL-10 expression by a variety of standard methods such as RT-PCR, western blots and immunohistochemistry. Likewise, the expression of their respective receptors was analysed by the same methods. Purified cancer cells were then tested for their sensitivity against chemotherapeutic agents such as e.g. etoposide, doxorubicin, oxaliplatin and apoptosis inducers such as TRAIL and CD95 ligand. The results are shown in the following Table 3.

**Table 3. Sensitization to death receptors- and chemotherapy-induced cell death**

| | | | | **Anti IL-4 treatment** | | |
|---|---|---|---|---|---|---|
| **Specimens** | **Number** | **IL-4 expression** | **IL-10 expression** | **Chemotherapy** | **TRAIL** | **CD95** |
| **Thyroid** | 75 | 75 | 75 | 2/20 | N.D. | 5/20 |
| **Colon** | 85 | 68 | 5 | 16/20 | 15/20 | 16/20 |
| **Gastric** | 21 | 14 | 10 (low) | 9/10 | 10/10 | 7/10 |
| **Breast** | 25 | 16 | 11 (low) | 8/10 | 6/10 | 9/10 |
| **Lung** | 9 | 5 | 1 (low) | 4/2 | 4/2 | 3/3 |
| **Prostate** | 12 | 10 | 1 | 4/5 | N.D. | 3/5 |
| **Pancreas** | 6 | 6 (low) | 6 (low) | N.D. | N.D. | N.D. |
| **Bladder** | 12 | 12 | 10 (low) | 3/4 | N.D. | 2/4 |
| **Liver** | 4 | 4 (low) | 4 (low) | N.D. | N.D. | N.D. |
| **Kidney** | 3 | 3 (low) | 3 (low) | N.D. | N.D. | N.D. |

As shown from the results in Table 3, it was surprisingly found that normally resistent primary tumour cells expressing IL-4 and/or IL-10 became sensitive against the tested chemotherapeutic agents and/or the pro-apoptotic agents when incubated in the presence of an IL-4 antibody such that more than 90% of the cells died in a couple of days. Particular significant sensitisation to death-receptors and chemotherapy-induced cell death was shown for colon, gastric, breast, lung, prostate and bladder cancer cells.

### Example 2

The data reported in this example reveal that purified colon cancer stem cells produce high levels of IL-4 and that the exposure of the cancer cells to neutralising antibodies against IL-4 sensitised cells to cytotoxic drug- and TRAIL-induced apoptosis. Further, the following data show that a combined treatment of colon tumours with chemotherapeutic agents and anti-IL-4 agents significantly reduces tumour outgrowth.

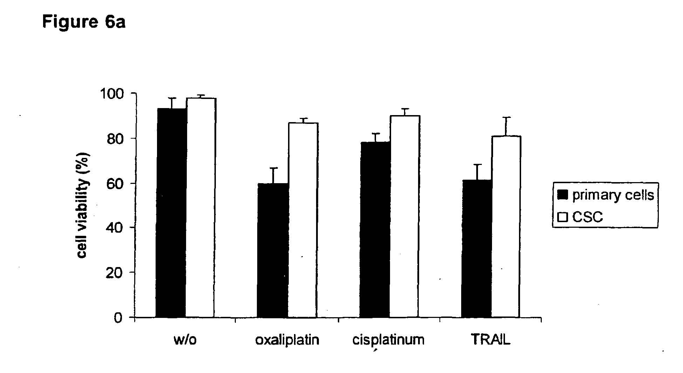

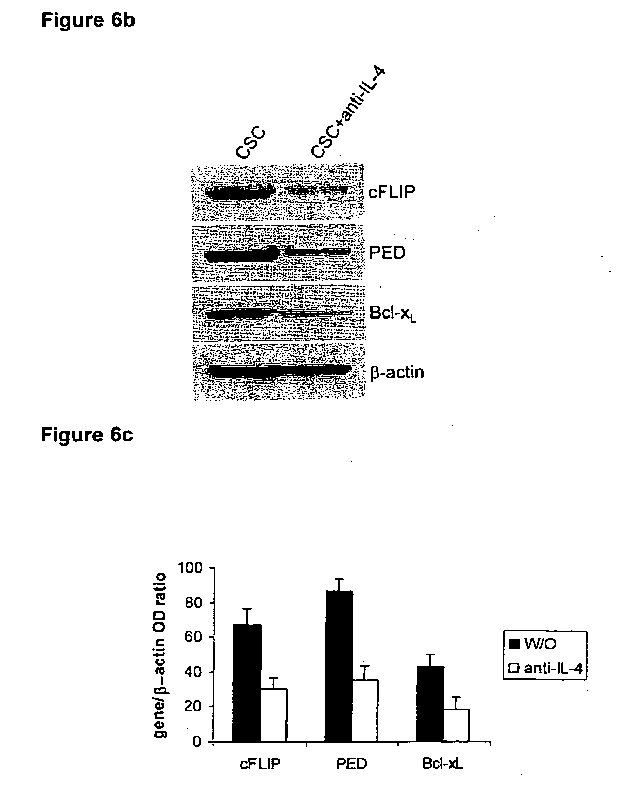

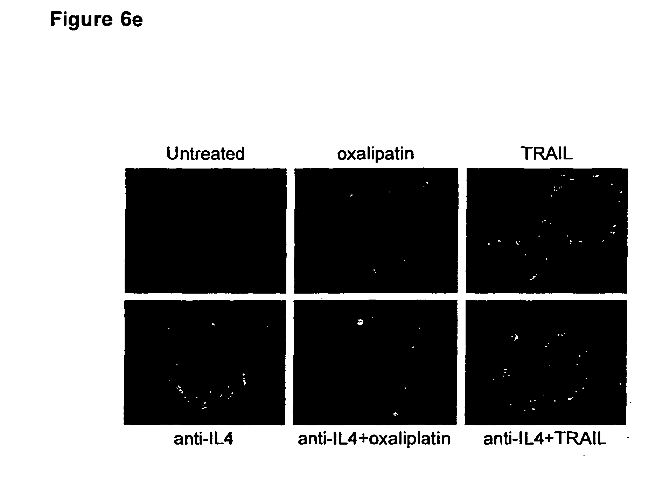

To investigate the sensitivity of colon CSC to chemotherapeutic drugs, the viability of colon CSC spheroids exposed to cisplatinum (300ng/ml) and oxaliplatin (100 µM) was measured, doses equivalent to those reached during cancer treatment *in vivo*. In addition, colon CSC were treated with the apoptosis-inducing death ligand TRAIL (200 ng/ml). Primary (adherent) cells from human colon cancer specimens showed some sensitivity *in vitro* to all three drugs tested, whereas colon CSC were significantly resistant, confirming that CSC are relatively inert to drug-induced apoptosis (Fig. 6a). This suggests that CSC might escape anti-tumour therapies and could be the underlying reason for chemotherapy inefficiency.

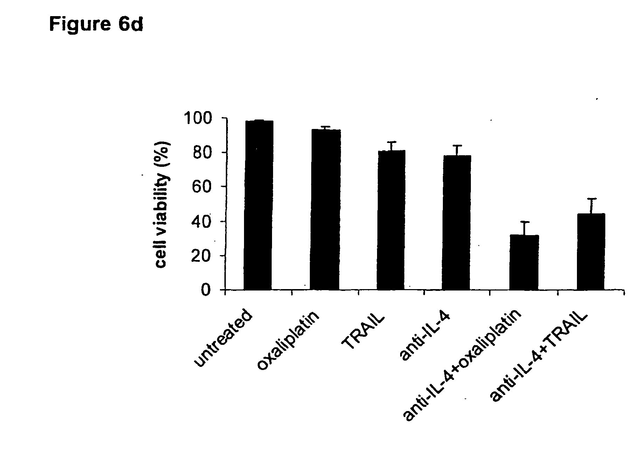

To formally prove that IL-4 production in colon CSC is responsible for upregulation of anti-apoptotic proteins and therefore therapy refractoriness, CSC were pre-treated for two days with IL-4-neutralising antibodies and then measured cell death and anti-apoptotic expression. Proteins levels of cFLIP, Bcl-xL and PED, anti-apoptotic proteins previously shown to be regulated by IL-4 in cancer, decreased by -two-fold in CSC exposed to anti-IL-4 (Fig. 6b-c). More important, following IL-4 blockade CSC cell death was significantly increased by the treatment with chemotherapeutic drugs or TRAIL (Fig. 6d-e).

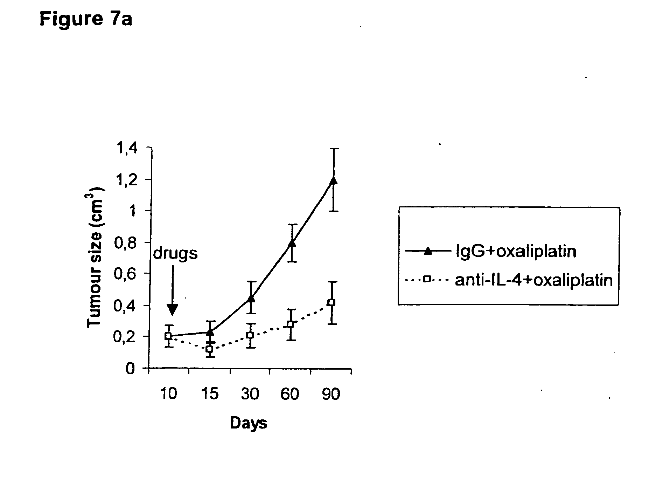

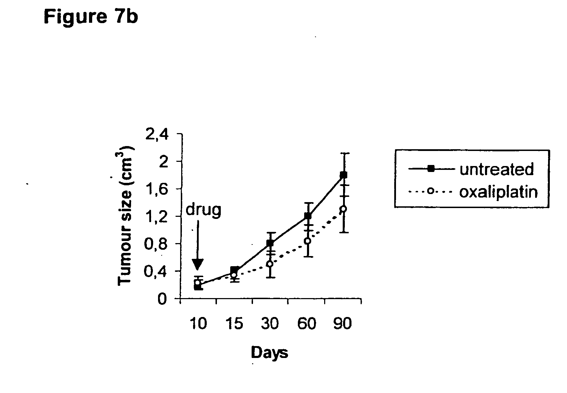

To directly demonstrate that IL-4 protects colon cancer generated by CSC from chemotherapeutic drugs, the effects of IL-4 neutralization *in vivo* were investigated. Tumours were allowed to grow for 10 days (size -0.2 cm³) and then treated intra-tumourally with neutralising antibodies against IL-4 or control IgG twice a week for 3 weeks. Although intraperitoneal (i.p.) treatment with oxaliplatin, once a week for 4 weeks, combined with control IgG reduced tumour size in mice, the efficacy of chemotherapy treatment was significantly enhanced by IL-4 neutralizing antibody (Fig. 7a and 7b).

### Example 3

### Construction of an IL4RIL13R-Fc fusion polypeptide

The signal-peptide and the extracellular domain of IL4-Receptor-alpha (aa1-aa231 of NCBI accession NP_000409) was fused N-terminally to the IL13-receptor alpha extracellular domain (aa27-aa343 of NCBI accession NP_001551) Two point mutations were introduced into the IL4R-alpha1-sequence (Gly2->Val2 and Cys207->Ser207) and a single point mutation was introduced into the IL13R-alpha1-sequence (Cys46->Ala46). The enumeration of the point mutations also refers to NCBI-database entries NP_000409 for IL4R-alpha1 and NP_001551 for IL13R-alpha1.

This IL4RIL13R protein-sequence was fused to the Fc-part of human IGHG1 (aa254-aa479 of NCBI accession AAH69020). Additionally, a flexible linker element and a Flexstreptag-II motif (SSSSSSAWSHPQFEK) was added C-terminally. The amino acid sequence of the resulting IL4RIL13R-Fc-construct as shown below was backtranslated into a synthetic DNA-sequence and its codon usage optimised for mammalian cell-based expression. Gene synthesis was done by ENTELECHON GmbH (Regensburg, Germany). The final expression cassette was subcloned into pCDNA4-HisMax-backbone, using the unique Hind-III- and Not-I-sites of the plasmid. 
SEQ ID NO: 1
SEQ IL4RIL13R-Fc.PRO
KEYWORD PROTEIN
ORIGIN
1 

61 

121 

181 

241 

301 

361 

421 

481 

541 

601 

661 

721 

781 
aa1-aa23: signal peptide
aa24-aa231: IL4R-alpha1 ECD
aa232-aa548: IL13R-alpha1 ECD
aa549-aa775: Fc part of IGHG1
aa786-aa790: Flexstreptag-II

Modifications of the IL4R-IL13R-Fc fusion polypeptide may be as follows:
- absence of the signal peptide or presence of a heterologous signal peptide;
- presence of a different, e.g. shortened IL-4R ECD, e.g. without or with different mutations, particularly point mutations,

- presence of a different effector domain,
- presence of a different Fc domain, and/or
- absence of the C-terminal purification domain (particularly for pharmaceutical applications).

### Example 4

### Construction of an IL4R-Fc fusion polypeptide

The signal-peptide and a shortened extracellular domain of IL4-Receptor-alpha (aa1-aa224 of NCBI accession NP_000409) was fused N-terminally to the Fc-part of human IGHG1 (aa250-aa479 of NCBI accession AAH69020). Two point mutations were introduced into the IL4R-alpha1-sequence (Gly2->Val2 and Cys207->Ser207). A single glycine was inserted inbetween the two domains and Lys251 of human IGHG1 in the hinge region was mutated to arginine. The enumeration of the described mutations also refer to NCBI-database entries NP_000409 for IL4R-alpha1 and NCBI accession AAH69020 for IGHG1).

Additionally, a flexible linker element and a Flexstreptag-II motif (SSSSSSAWSHPQFEK) was added C-terminally. The amino acid sequence of the resulting IL4R-Fc-construct as shown below was backtranslated into a synthetic DNA-sequence and its codon usage optimised for mammalian cell-based expression. Gene synthesis was done by ENTELECHON GmbH (Regensburg, Germany). The final expression cassette was subcloned into pCDNA4-HisMax-backbone, using the unique Hind-III- and Not-I-sites of the plasmid.

SEQ ID NO:2
SEQ          IL4RA-Fc.PRO
KEYWORD      PROTEIN

COLOURS
    sequence = 1

features = 0
ORIGIN
1        MVWLCSGLLF PVSCLVLLQV ASSGNMKVLQ EPTCVSDYMS ISTCEWKMNG
PTNCSTELRL
61       LYQLVFLLSE AHTCIPENNG GAGCVCHLLM DDVVSADNYT LDLWAGQQLL
WKGSFKPSEH
121      VKPRAPGNLT VHTNVSDTLL LTWSNPYPPD NYLYNHLTYA VNIWSENDPA
DFRIYNVTYL
181      EPSLRIAAST LKSGISYRAR VRAWAQSYNT TWSEWSPSTK WHNSGSRSCD
KTHTCPPCPA
241      PELLGGPSVF LFPPKPKDTL MISRTPEVTC VVVDVSHEDP EVKFNWYVDG
VEVHNAKTKP
301      REEQYNSTYR VVSVLTVLHQ DWLNGKEYKC KVSNKALPAP IEKTISKAKG
QPREPQVYTL
361      PPSREEMTKN QVSLTCLVKG FYPSDIAVEW ESNGQPENNY KTTPPVLDSD
GSFFLYSKLT
421      VDKSRWQQGN VFSCSVMHEA LHNHYTQKSL SLSPGSSSSS SAWSHPQFEK

Aa1-aa23: IL4R-alpha1 signal peptide
Aa24-aa224: IL4R-alpha1 ECD
Aa225-aa455: Fc part of IGHG1
Aa456-aa470: Flexstreptag-II

Modifications of the shortened IL-4R fusion polypeptide may be as follows:
- absence of a signal peptide or presence of a heterologous signal peptide;
- presence of the different, e.g. shortened IL-4R ECD, e.g. without or with different mutations, particularly point mutations,
- presence of a different Fc domain,
- a different fusion region between the IL-4R ECD and the Fc domain, e.g.
   deletion of one or more amino acids of the sequence RSC (positions 227-229), and/or
- absence of the C-terminal purification domain (particularly for pharmaceutical applications).

### Example 5

### Expression and Purification of IL4-binding proteins, IL4R-Fc and IL4R-IL13R-Fc

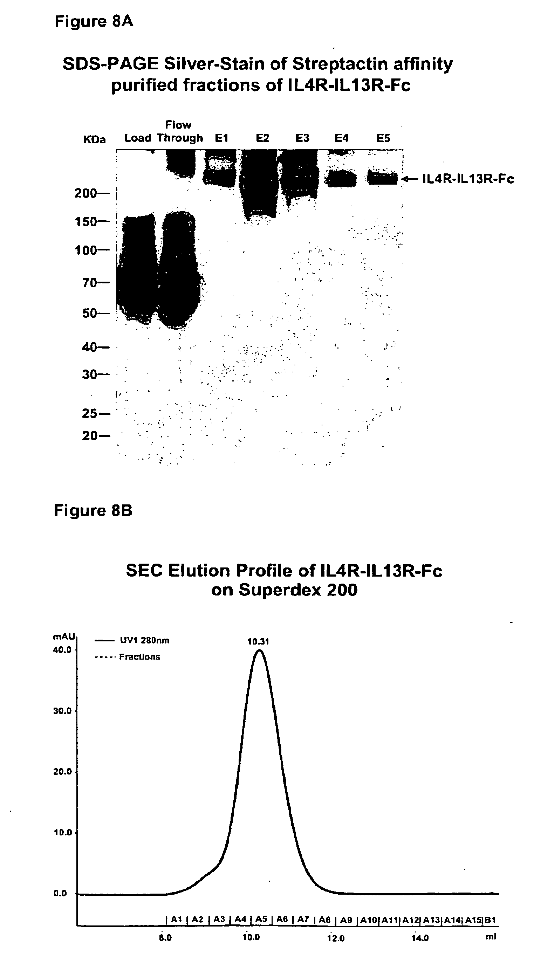

Hek 293T cells grown in DMEM + GlutaMAX (GibCo) supplemented with 10 % FBS, 100 units/ml Penicillin and 100 µg/ml Streptomycin were transiently transfected with plasmids encoding IL4R-Fc and IL4R-IL13R-Fc, respectively. Cell culture supernatants containing recombinant proteins were harvested three days post transfection and clarified by centrifugation at 300 g followed by filtration through a 0.22 µm sterile filter. For affinity purification Streptactin Sepharose was packed to a column (gel bed 1 ml), equilibrated with 15 ml buffer W (100 mM Tris-HCl, 150 mM NaCl pH 8.0) and the respective cell culture supernatant was applied to the column with a flow rate of 4 ml/min. Subsequently, the column was washed with buffer W and bound IL4R-Fc or IL4R-IL13R-Fc was eluted stepwise by addition of 6 x 1 ml buffer E (100 mM Tris HCl, 150 mM NaCl, 2.5 mM Desthiobiotin pH 8.0). The protein amount of the eluate fractions was quantified and peak fractions were concentrated by ultrafiltration and further purified by size exclusion chromatography (SEC). An SDS-PAGE of the Streptactin affinity purification of IL4R-IL13R-Fc followed by Silver staining is shown in Figure 8A.

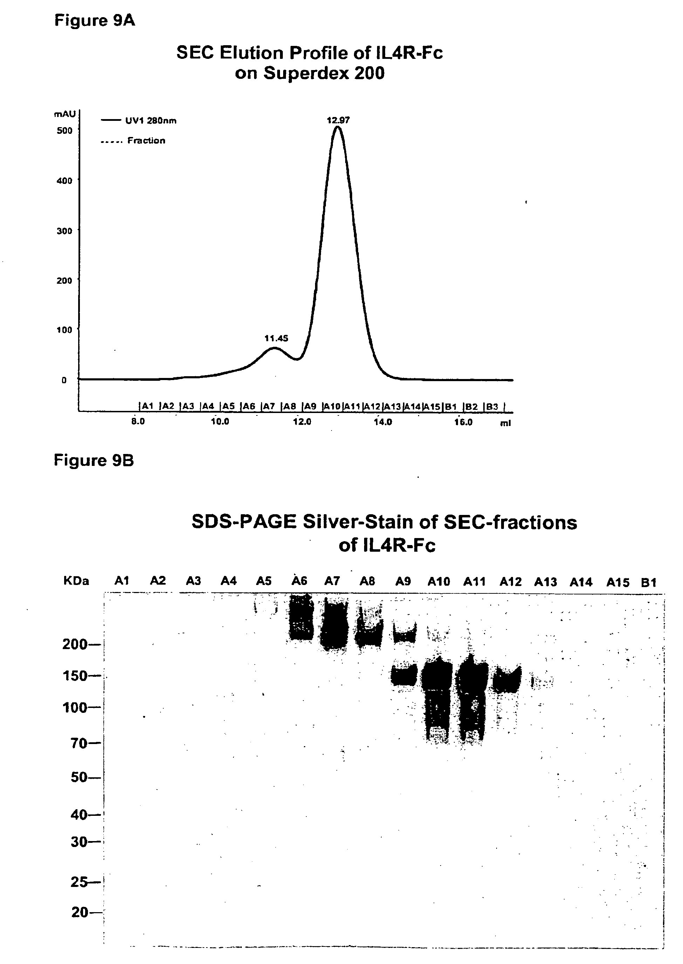

SEC was performed on a Superdex 200 column using an Akta chromatography system (GE-Healthcare). The column was equilibrated with phosphate buffered saline and the concentrated, streptactin purified IL4R-Fc or IL4R-IL13R-Fc, respectively, were loaded onto the SEC column at a flow rate of 0.5 ml/min. The elution profile monitored by absorbance at 280 nm showed a prominent protein peak at 10.31 ml for IL4R-IL13R-Fc (Figure 8B) and 12.97 ml for IL4R-Fc (Figure 9A). SEC fractions for IL4R-Fc were additionally analysed under denaturing conditions by SDS-PAGE and silver staining (Figure 9B).

For determination of the apparent molecular weight under native conditions a Superdex 200 column was loaded with standard proteins of known molecular weight. Based on the elution volume of the standard proteins a calibration curve was calculated and the apparent molecular weight of purified IL4R-Fc was determined to be 137 KDa which fits well to the molecular weight observed by SDS-PAGE. The theoretical molecular weight based on the amino acid sequence of IL4R-Fc is 52.8 Kda for the monomeric protein. Based on the biochemical analysis IL4R-Fc very likely is expressed as a protein dimer.

For IL4R-IL13R-Fc the apparent molecular weight based on SEC was calculated to be about 600 KDa. Based on SDS-Page analysis the protein runs as a single band with about 250Kda. The theoretical molecular weight based on the amino acid sequence of IL4R-IL13R-Fc is 87.7 KDa. In principle the construction of the molecule should result in a stable dimeric protein with a theoretical molecular weight of about 180Kda. The high apparent molecular weight seen by SEC therefore either indicates an unusual behavior in SEC or further oligomerisation of the protein.

### IL4-pull down assay

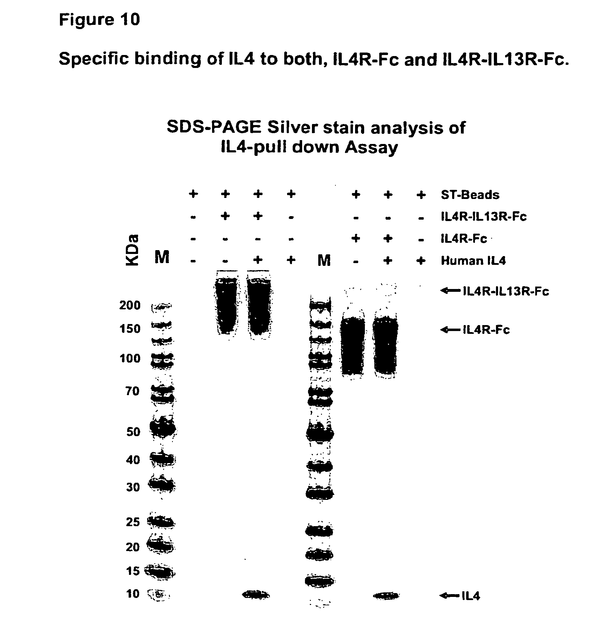

To test for specific IL4 binding of IL4R-Fc and IL4R-IL13R-Fc, 4µg of both proteins, respectively, were immobilized to Streptactin Sepharose via their Strep-Tag. The immobilized proteins were subsequently incubated for 60 min with 400 ng of recombinantly expressed human Interleukin4 (IL4) in a total volume of 400 µl phosphate buffered saline. Subsequently the beads were washed and bound proteins were specifically eluted with desthiobiotin in a total volume of 40 µl elution buffer. Eluted proteins were finally analysed via SDS-PAGE and Silver staining. As shown in Figure 10 both IL4R-Fc and IL4R-IL13R-Fc show specific binding of human IL4 indicated by the presence of IL4 protein (12Kda) that could not be seen in control experiments.

### Example 6

### In vitro efficacy on Cancer stem cells and primary tumor cells

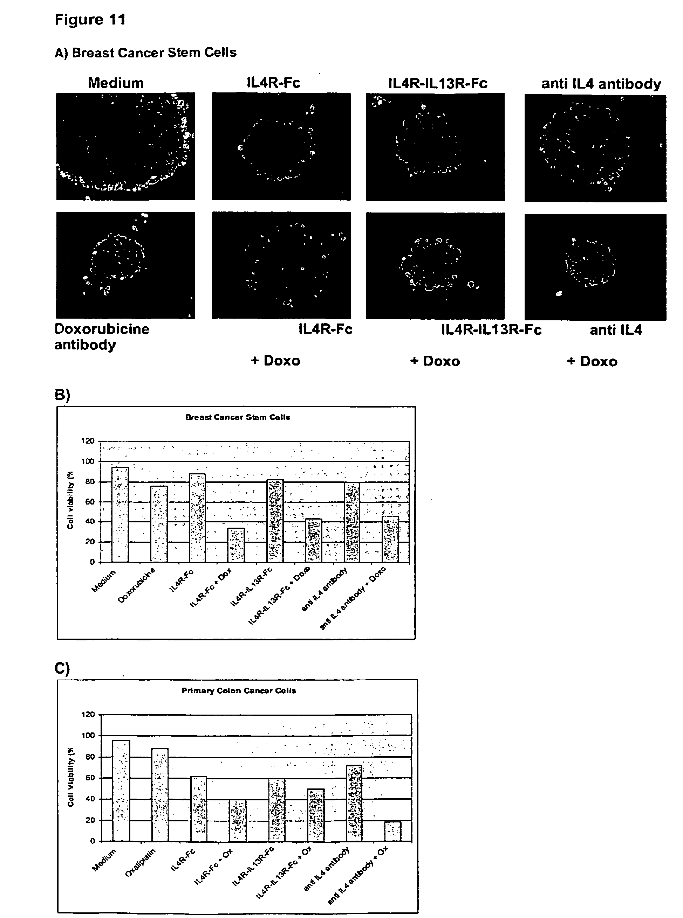

To test for the ability of IL4R-Fc and IL4R-IL13R-Fc to induce apoptosis, both proteins were added to the growth medium of breast cancer stem cells either alone or in combination with doxorubicin. Figure 11A shows the immunofluorescence analysis of breast cancer spheres pre-treated with PBS (w/o) or 10 µg of IL4R-Fc, IL4R-IL13R-Fc or anti IL4-antibody for 24 hrs and successively exposed for another 24 hrs to 5 µM doxorubicin. The cells were stained with orange acridine/ethidium bromide (red: dead cells; green: viable cells). In comparison with the single treatment (Doxorubicin alone) the combination of doxorubicin with either IL4R-Fc or IL4R-IL13R-Fc, respectively, clearly increased the amout of apoptotic breast cancer stem cells. A cell count discriminating apoptotic and living cells, subsequently plotted for the percentage of cell viability also demonstrates the efficacy of the combination treatmant for the induction of apoptosis (Figure 11 B). Importantly both IL4R-Fc and IL4R-IL13R-Fc are able to sensitise breast cancer stem cells for doxorubicin induced apoptosis in the same range as shown for an IL4 specific antibody, that was used as a positive control in this experiment.

On primary colon cancer cells the IL4R-Fc and IL4R-IL13R-Fc constructs were tested in combination with oxaliplatin treatment. Primary colon cancer cells pre-treated with PBS (w/o) or 10 µg of IL4R-Fc, IL4R-IL13R-Fc or anti IL4-antibody for 24 hrs and successively exposed for another 24 hrs to 100 µM oxaliplatin. The graphs show the percentage of cell viability measured by MTS analysis (CellTiter 96, Aquos, Promega ). As shown in Figure 11C, both constructs are able to sensitze primary colon cancer cells for oxaliplatin induced apoptosis, indicated by a reduced cell viability in comparison with oxaliplatin treatment alone.

## Claims

1. An in vitro method for diagnosing a cancer type comprising the steps:
(a) providing a sample from an epithelial tumour comprising tumour cells,
(b) determining the expression of at least one anti-apoptotic cytokine in said tumour cells, and
(c) classifying the epithelial tumour as a non-cytokine expressing tumour or as a cytokine expressing tumour,
wherein the anti-apoptotic cytokine is IL-4 and/or IL-10,
wherein the epithelial tumour is classified as an IL-4 expressing, an IL-4 non-expressing tumour, an IL-10 expressing, an IL-10 non-expressing tumour, an IL-4 and IL-10 expressing tumour, or as a non-IL-4 and a non-IL-10 expressing tumour.

2. The in vitro method according to claim 1, wherein the epithelial tumour is selected from the group of thyroid, breast, prostate, bladder, colon, gastric, pancreas, kidney, liver and lung cancer.

3. The in vitro method according to claim 2 wherein the tumour preferably is a colon, gastric, breast, lung, bladder, or prostate cancer.

4. The in vitro method according to any of the preceding claims, wherein the tumour cells are primary tumour cells and/or cancer stem cells.

5. The in vitro method according to any of the preceding claims, wherein detecting the anti-apoptotic cytokine expression in the tumour cells comprises a detection on the protein level and/or on the nucleic acid level, preferably:
(a) a detection on the protein level which comprises the detection of the anti-apoptotic cytokine, preferably with immunochemical and/or mass spectrometric methods, or
(b) a determination on nucleic acid level which comprises the determination of anti-apoptotic cytokine mRNA expression levels with nucleic acid hybridization and optionally amplification methods, preferably with RT-PCR methods.

6. The in vitro method according to any of the preceding claims, further comprising the steps of
(d) determining the sensitivity of the cells of a cytokine expressing tumour against at least one chemotherapeutic or pro-apoptotic agent in the presence and/or in the absence of an antagonist of said expressed cytokine, and/or its receptor, preferably determining a chemotherapeutic or pro-apoptotic agent against which the cells of the cytokine-expressing tumour are sensitive, and
(e) optionally selecting a cancer type-specific treatment, preferably selecting a treatment comprising the administration of a combination of a cytokine neutralizing agent and a chemotherapeutic or pro-apoptotic agent, wherein the cytokine neutralizing agent is an IL-4 and/or IL-10 neutralizing agent, wherein the cytokine neutralizing agent is an IL-4 or IL-4 and IL-10 neutralizing agent.

7. The in vitro method according to claim 6 wherein the chemotherapeutic agent is selected from antimetabolites, DNA-fragmenting agents, DNA-crosslinking agents, intercalating agents, protein synthesis inhibitors, topoisomerase I and II inhibitors, microtubule-directed agents, kinase inhibitors, hormones and hormone antagonists or wherein the pro-apoptotic agent is selected from TRAIL and CD95 ligand.

8. The in vitro method according to claim 7 wherein the chemotherapeutic agent is selected from cisplatin, carboplatin and oxaliplatin.

9. The in vitro method according to any one of claims 6 to 8, wherein the cytokine neutralizing agent is an anti-IL-4 antibody and/or an anti-IL-10 antibody or an antigen-binding fragment thereof.

10. The in vitro method according to claim 9, wherein the anti-IL-4 antibody is an antibody derived from the hybridoma cell ECACC 93100620 or an antigen-binding fragment thereof.

11. The in vitro method according to any of claims 6 to 8, wherein the cytokine neutralizing agent is a soluble IL-4 receptor polypeptide or fusion polypeptide.

12. A combination of
(i) at least one cytokine neutralizing agent and
(ii) at least a chemotherapeutic or pro-apoptotic agent which pro-apoptotic agent is selected from TRAIL or CD95L for use in a method for the treatment of eptithelial tumours that have become refractory to chemotherapeutic agents, wherein the cytokine neutralizing agent is an IL-4 neutralizing agent inhibiting the expressed cytokine protein activity.

13. A combination of
(i) at least one cytokine neutralizing agent and
(ii) at least a chemotherapeutic or pro-apoptotic agent which pro-apoptotic agent is selected from TRAIL or CD95L for use in a method for the treatment of cytokine-expressing epithelial tumour, wherein the cytokine neutralizing agent is an IL-4 neutralizing agent inhibiting the expressed cytokine protein activity.

14. The combination of claim 13 for use in a method as defined in claim 13 in combination with surgery and/or irradiation therapy.

15. A combination of
(i) at least one cytokine neutralizing agent and
(ii) at least a chemotherapeutic or pro-apoptotic agent which pro-apoptotic agent is selected from TRAIL or CD95L for use in a method for the treatment of a cytokine-expressing epithelial tumour wherein the administration of (i) and (ii) is started simultaneously; or wherein the administration of (i) and (ii) is started stepwise, wherein the cytokine neutralizing agent is an IL-4 neutralizing agent inhibiting the expressed cytokine protein activity.

16. The combination according to claim 15, for use in a method as defined in claim 15, wherein the start of administration of (i) is ≥ 1 week before (ii) or wherein the start of administration of (ii) is ≥ 1 week before (i).

17. A human soluble IL-4 receptor alpha (NCBI accession number NP_000409) which C-terminally ends at amino acid 230, 229, 228, 227, 226, 225 or 224 for use in a method for the treatment of epithelial tumours.

18. A soluble IL-4 receptor polypeptide comprising a C-terminally shortened extracellular IL-4 receptor domain comprising at least one further domain wherein the further domain is an IL-13 receptor extracellular domain for use in a method for the treatment of epithelial tumours.

## Patentansprüche

1. In vitro-Verfahren zur Diagnose einer Krebsart, umfassend die Schritte:
(a) Bereitstellen einer Probe aus einem epithelialen Tumor, umfassend Tumorzellen,
(b) Bestimmen der Expression mindestens eines antiapoptotischen Cytokins in den Tumorzellen und
(c) Klassifizieren des epithelialen Tumors als ein nicht-Cytokin-exprimierender Tumor oder als ein Cytokin-exprimierender Tumor,
wobei das antiapoptotische Cytokin IL-4 und/oder IL-10 ist, wobei der epitheliale Tumor als ein IL-4-exprimierender, ein nicht-IL-4-exprimierender Tumor, ein IL-10-exprimierender, ein nicht-IL-10-exprimierender Tumor, ein IL-4- und IL-10-exprimierender Tumor oder als ein nicht-IL-4- und nicht-IL-10-exprimierender Tumor klassifiziert wird.

2. In vitro-Verfahren gemäß Anspruch 1, wobei der epitheliale Tumor aus der Gruppe aus Schilddrüsen-, Brust-, Prostata-, Blasen-, Dickdarm-, Magen-, Pankreas-, Nieren-, Leber- und Lungenkrebs ausgewählt ist.

3. In vitro-Verfahren gemäß Anspruch 2, wobei der Tumor bevorzugt ein Dickdarm-, Magen-, Brust-, Lungen-, Blasen- oder Prostatakrebs ist.

4. In vitro-Verfahren gemäß einem der vorhergehenden Ansprüche, wobei die Tumorzellen Primärtumorzellen und/oder Krebsstammzellen sind.

5. In vitro-Verfahren gemäß einem der vorhergehenden Ansprüche, wobei das Detektieren der antiapoptotischen Cytokinexpression in den Tumorzellen eine Detektion auf dem Proteinlevel und/oder auf dem Nukleinsäurelevel umfasst, bevorzugt:
(a) eine Detektion auf dem Proteinlevel, die die Detektion des antiapoptotischen Cytokins umfasst, bevorzugt mit immunchemischen und/oder massenspektrometrischen Verfahren, oder
(b) eine Bestimmung auf dem Nukleinsäurelevel, die die Bestimmung von Expressionsleveln von antiapoptotischer Cytokin-mRNA mit Nukleinsäurehybridisierung und gegebenenfalls Amplifikationsverfahren, bevorzugt mit RT-PCR-Verfahren, umfasst.

6. In vitro-Verfahren gemäß einem der vorhergehenden Ansprüche, weiterhin umfassend die Schritte
(d) Bestimmen der Empfindlichkeit der Zellen eines Cytokin-exprimierenden Tumors gegenüber mindestens einem chemotherapeutischen oder proapoptotischen Mittel in Anwesenheit und/oder in Abwesenheit eines Antagonisten des exprimierten Cytokins und/oder seines Rezeptors, bevorzugt Bestimmen eines chemotherapeutischen oder proapoptotischen Mittels, gegenüber welchem die Zellen des Cytokin-exprimierenden Tumors empfindlich sind und
(e) gegebenenfalls Auswählen einer Krebsart-spezifischen Behandlung, bevorzugt Auswählen einer Behandlung, die die Verabreichung einer Kombination eines Cytokin-neutralisierenden Mittels und eines chemotherapeutischen oder proapoptotischen Mittels umfasst, wobei das Cytokin-neutralisierende Mittel ein IL-4- und/oder IL-10-neutralisierendes Mittel ist, wobei das Cytokin-neutralisierende Mittel ein IL-4- oder ein IL-4- und IL-10-neutralisierendes Mittel ist.

7. In vitro-Verfahren gemäß Anspruch 6, wobei das chemotherapeutische Mittel aus Antimetaboliten, DNA-Fragmentierungsmitteln, DNA-Vernetzungsmitteln, interkalierenden Mitteln, Proteinsyntheseinhibitoren, Topoisomerase I- und II-Inhibitoren, auf Mikrotubuli gerichteten Mitteln, Kinaseinhibitoren, Hormonen und Hormon-Antagonisten ausgewählt ist, oder wobei das proapoptotische Mittel aus TRAIL und CD95-Ligand ausgewählt ist.

8. In vitro-Verfahren gemäß Anspruch 7, wobei das chemotherapeutische Mittel aus Cisplatin, Carboplatin und Oxaliplatin ausgewählt ist.

9. In vitro-Verfahren gemäß einem der Ansprüch 6 bis 8, wobei das Cytokin-neutralisierende Mittel ein Anti-IL-4-Antikörper und/oder ein Anti-IL-10-Antikörper oder ein Antigen-bindendes Fragment davon ist.

10. In vitro-Verfahren gemäß Anspruch 9, wobei der Anti-IL-4-Antikörper ein Antikörper ist, der aus der Hybridomzelle ECACC 93100620 stammt, oder ein Antigen-bindendes Fragment davon.

11. In vitro-Verfahren gemäß einem der Ansprüch 6 bis 8, wobei das Cytokin-neutralisierende Mittel ein Polypeptid oder Fusionspolypeptid von löslichem IL-4-Rezeptor ist.

12. Kombination von
(i) mindestens einem Cytokin-neutralisierenden Mittel und
(ii) mindestens einem chemotherapeutischen oder proapoptotischen Mittel, wobei das proapoptotische Mittel aus TRAIL oder CD95L ausgewählt ist, zur Verwendung in einem Verfahren zur Behandlung von epithelialen Tumoren, die gegenüber chemotherapeutischen Mitteln refraktär geworden sind, wobei das Cytokin-neutralisierende Mittel ein IL-4-neutralisierendes Mittel ist, das die Aktivität von exprimiertem Cytokinprotein inhibiert.

13. Kombination von
(i) mindestens einem Cytokin-neutralisierenden Mittel und
(ii) mindestens einem chemotherapeutischen oder proapoptotischen Mittel, wobei das proapoptotische Mittel aus TRAIL oder CD95L ausgewählt ist, zur Verwendung in einem Verfahren zur Behandlung eines Cytokin-exprimierenden epithelialen Tumors, wobei das Cytokin-neutralisierende Mittel ein IL-4-neutralisierendes Mittel ist, das die Aktivität von exprimiertem Cytokinprotein inhibiert.

14. Kombination nach Anspruch 13 zur Verwendung in einem Verfahren, wie in Anspruch 13 definiert, in Kombination mit einer Operation und/oder Strahlentherapie.

15. Kombination von
(i) mindestens einem Cytokin-neutralisierenden Mittel und
(ii) mindestens einem chemotherapeutischen oder proapoptotischen Mittel, wobei das proapoptotische Mittel aus TRAIL oder CD95L ausgewählt ist, zur Verwendung in einem Verfahren zur Behandlung eines Cytokin-exprimierenden epithelialen Tumors, wobei mit der Verabreichung von (i) und (ii) gleichzeitig begonnen wird; oder wobei mit der Verabreichung von (i) und (ii) schrittweise begonnen wird, wobei das Cytokin-neutralisierende Mittel ein IL-4-neutralisierendes Mittel ist, das die Aktivität von exprimiertem Cytokinprotein inhibiert.

16. Kombination gemäß Anspruch 15 zur Verwendung in einem Verfahren, wie in Anspruch 15 definiert, wobei der Beginn der Verabreichung von (i) ≥ 1 Woche vor (ii) ist, oder wobei der Beginn der Verabreichung von (ii) ≥ 1 Woche vor (i) ist.

17. Humaner löslicher IL-4-Rezeptor alpha (NCBI Zugangsnummer NP_000409), der C-terminal an der Aminosäure 230, 229, 228, 227, 226, 225 oder 224 endet, zur Verwendung in einem Verfahren zur Behandlung von epithelialen Tumoren.

18. Polypeptid des löslichen IL-4-Rezeptors, umfassend eine C-terminal verkürzte extrazelluläre IL-4-Rezeptordomäne, die mindestens eine weitere Domäne umfasst, wobei die weitere Domäne eine extrazelluläre IL-13-Rezeptordomäne ist, zur Verwendung in einem Verfahren zur Behandlung von epithelialen Tumoren.

## Revendications

1. Procédé *in vitro* destiné à diagnostiquer un type de cancer comprenant les étapes consistant à :
(a) fournir un échantillon provenant d'une tumeur épithéliale comprenant des cellules tumorales,
(b) déterminer l'expression d'au moins une cytokine anti-apoptotique dans lesdites cellules tumorales, et
(c) classifier la tumeur épithéliale comme tumeur n'exprimant pas de cytokine ou comme tumeur exprimant une cytokine,
dans lequel la cytokine anti-apoptotique est une IL-4 et/ou une IL-10,
la tumeur épithéliale étant classifiée comme tumeur exprimant une IL-4, n'exprimant pas d'IL-4, exprimant une IL-10, n'exprimant pas d'IL-10, exprimant une IL-4 et une IL-10, ou n'exprimant ni une IL-4 ni une IL-10.

2. Procédé *in vitro* selon la revendication 1, dans lequel la tumeur épithéliale est choisie dans le groupe comprenant un cancer de la thyroïde, du sein, de la prostate, de la vessie, du colon, de l'estomac, du pancréas, du rein, du foie et des poumons.

3. Procédé *in vitro* selon la revendication 2, dans lequel la tumeur est de préférence un cancer du colon, de l'estomac, du sein, des poumons, de la vessie, ou de la prostate.

4. Procédé *in vitro* selon l'une quelconque des revendications précédentes, dans lequel les cellules tumorales sont des cellules tumorales primaires et/ou des cellules souches cancéreuses.

5. Procédé *in vitro* selon l'une quelconque des revendications précédentes, dans lequel la détection de l'expression de cytokines anti-apoptotiques dans les cellules tumorales comprend la détection au niveau des protéines et/ou au niveau des acides nucléiques, de préférence :
(a) une détection au niveau des protéines comprenant la détection de la cytokine anti-apoptotique, de préférence par des procédés immunochimiques et/ou de spectrométrie de masse, ou
(b) une détermination au niveau des acides nucléiques comprenant la détermination des niveaux d'expression de l'ARNm de la cytokine anti-apoptotique par des procédés d'hybridation d'acides nucléiques et éventuellement d'amplification, de préférence par des procédés de RT-PCR.

6. Procédé *in vitro* selon l'une quelconque des revendications précédentes, comprenant en outre les étapes consistant à :
(d) déterminer la sensibilité des cellules d'une tumeur exprimant une cytokine à au moins un agent chimiothérapeutique ou pro-apoptotique en présence et/ou en l'absence d'un antagoniste de ladite cytokine exprimée, et/ou de son récepteur, de préférence déterminer un agent chimiothérapeutique ou pro-apoptotique auquel sont sensibles les cellules de la tumeur exprimant la cytokine, et
(e) éventuellement choisir un traitement spécifique du type de cancer, de préférence choisir un traitement comprenant l'administration d'une combinaison d'un agent neutralisant les cytokines et d'un agent chimiothérapeutique ou pro-apoptotique, dans lequel l'agent neutralisant les cytokines est un agent neutralisant l'IL-4 et/ou l'IL-10, dans lequel l'agent neutralisant les cytokines est un agent neutralisant l'IL-4 ou l'IL-4 et l'IL-10.

7. Procédé *in vitro* selon la revendication 6, dans lequel l'agent chimiothérapeutique est choisi parmi des antimétabolites, des agents fragmentant l'ADN, des agents réticulant l'ADN, des agents intercalants, des inhibiteurs de synthèse protéique, des inhibiteurs des topoisomérases I et II, des agents dirigés contre les microtubules, des inhibiteurs de kinases, des hormones et des antagonistes d'hormones ou dans lequel l'agent pro-apoptotique est choisi parmi les ligands TRAIL et CD95.

8. Procédé *in vitro* selon la revendication 7, dans lequel l'agent chimiothérapeutique est choisi parmi le cisplatine, le carboplatine et l'oxaliplatine.

9. Procédé *in vitro* selon l'une quelconque des revendications 6 à 8, dans lequel l'agent neutralisant les cytokines est un anticorps anti-IL-4 et/ou un anticorps anti-IL-10 ou un fragment de ceux-ci se liant à un antigène.

10. Procédé *in vitro* selon la revendication 9, dans lequel l'anticorps anti-IL-4 est un anticorps dérivé de la lignée cellulaire d'hybridomes ECACC 93100620 ou un fragment de celui-ci se liant à un antigène.

11. Procédé *in vitro* selon l'une quelconque des revendications 6 à 8, dans lequel l'agent neutralisant les cytokines est un polypeptide récepteur soluble de l'IL-4 ou un polypeptide de fusion.

12. Combinaison de :
(i) au moins un agent neutralisant les cytokines et
(ii) au moins un agent chimiothérapeutique ou pro-apoptotique, ledit agent pro-apoptotique étant choisi parmi les TRAIL ou CD95L, destinée à être utilisée dans un procédé destiné au traitement de tumeurs épithéliales devenues réfractaires aux agents chimiothérapeutiques, dans lequel l'agent neutralisant les cytokines est un agent neutralisant l'IL-4 inhibant l'activité de la protéine cytokine exprimée.

13. Combinaison de :
(i) au moins un agent neutralisant les cytokines et
(ii) au moins un agent chimiothérapeutique ou pro-apoptotique, ledit agent pro-apoptotique étant choisi parmi les TRAIL ou CD95L, destinée à être utilisée dans un procédé destiné au traitement d'une tumeur épithéliale exprimant les cytokines, dans lequel l'agent neutralisant les cytokines est un agent neutralisant l'IL-4 inhibant l'activité de la protéine cytokine exprimée.

14. Combinaison selon la revendication 13 destinée à être utilisée dans un procédé tel que défini dans la revendication 13 en combinaison avec une chirurgie et/ou une radiothérapie.

15. Combinaison de :
(i) au moins un agent neutralisant les cytokines et
(ii) au moins un agent chimiothérapeutique ou pro-apoptotique, ledit agent pro-apoptotique étant choisi parmi les TRAIL ou CD95L, destinée à être utilisée dans un procédé destiné au traitement d'une tumeur épithéliale exprimant les cytokines, dans lequel les administrations de (i) et de (ii) sont débutées simultanément ; ou dans lequel les administrations de (i) et de (ii) sont débutées l'une après l'autre, dans lequel l'agent neutralisant les cytokines est un agent neutralisant l'IL-4 inhibant l'activité de la protéine cytokine exprimée.

16. Combinaison selon la revendication 15, destinée à être utilisée dans un procédé tel que défini dans la revendication 15, dans lequel le début de l'administration de (i) est ≥ 1 semaine avant (ii) ou dans lequel le début de l'administration de (ii) est ≥ 1 semaine avant (i).

17. Sous-unité alpha du récepteur de l'IL-4 soluble humaine (numéro d'enregistrement NCBI : NP_000409) se terminant à l'extrémité C-terminale par l'acide aminé 230, 229, 228, 227, 226, 225 ou 224, destiné à être utilisé dans un procédé de traitement de tumeurs épithéliales.

18. Polypeptide du récepteur de l'IL-4 soluble comprenant un domaine du récepteur de l'IL-4 extracellulaire raccourci au niveau de l'extrémité C-terminale comprenant au moins un autre domaine, l'autre domaine étant un domaine extracellulaire du récepteur de l'IL-13 destiné à être utilisé dans un procédé de traitement de tumeurs épithéliales.
